# EUROPÄISCHE PATENTSCHRIFT

(11) **EP 2 087 127 B1**
(45) Veröffentlichungstag und Bekanntmachung des Hinweises auf die Patenterteilung: **18.04.2012**
(21) Anmeldenummer: 07856445.7
(22) Anmeldetag: 07.12.2007
(51) Int. Cl.: C12P 33/00, C12P 41/00

(54) **VERFAHREN ZUR ENANTIOSELEKTIVEN ENZYMATISCHEN REDUKTION VON SECODIONDERIVATEN UNTER KONTINUIERLICHER COFAKTOR REGENERIERUNG**
PROCESS FOR THE ENANTIOSELECTIVE ENZYMATIC REDUCTION OF SECODIONE DERIVATIVES VIA CONTINUOUS COFACTOR REGENERATION
PROCÉDÉ DE RÉDUCTION ENZYMATIQUE ÉNANTIOSÉLECTIVE DE DÉRIVÉS DE SECODIONE PAR RÉGÉNÉRATION DE COFACTEUR EN CONTINU

(30) Priorität: 07.12.2006 AT 20272006
(43) Veröffentlichungstag der Anmeldung: 12.08.2009
(60) Teilanmeldung: 11177932.8
(73) Patentinhaber: IEP GmbH, 65203 Wiesbaden (DE)
(72) Erfinder: GUPTA, Antje, 65207 Wiesbaden (DE); TSCHENTSCHER, Anke, 65347 Eltville-Hattenheim (DE); BOBKOVA, Maria, 65207 Wiesbaden (DE)
(74) Vertreter: Schwarz, Albin
(86) Internationale Anmeldenummer: PCT/EP2007/010640
(87) Internationale Veröffentlichungsnummer: WO 2008/068030

(56) Entgegenhaltungen:
- WO-A-2006/087235
- SZENTIRMAI, A. ET AL.: "Properties of hydroxysteroid oxidoreductase isolated from yeast" ACTA MICROBIOLOGICA ACADEMIAE SCIENTIARUM HUNGARICAE, Bd. 22, Nr. 4, 1975, Seiten 463-470, XP008095188 in der Anmeldung erwähnt

### SEQUENCE LISTING

<110> IEP GmbH
<120> verfahren zur enantioselektiven enzymatischen Reduktion von Secodionderivaten
<130> I 11005
<160> 42
<170> PatentIn version 3.3
<210> 1
   <211> 252
   <212> PRT
   <213> Chloroflexus auratiacus DSM 635
<400> 1
   Met Glu Pro Pro Phe Ile Gly Lys Val Ala Leu Val Thr Gly Ala Ala
   1               5                   10                  15
   Ala Gly Ile Gly Arg Ala Ser Ala Leu Ala Phe Ala Arg Glu Gly Ala
               20                  25                  30
   Lys Val Val Val Ala Asp Val Asn Val Glu Gly Gly Glu Glu Thr Ile
           35                  40                  45
   Ala Leu Cys Arg Ala Leu Asn Thr Asp Ala Met Phe Val Arg Cys Asp
       50                  55                  60
   Val Ser Gln Arg Asp Glu Val Glu Arg Leu Ile Ala Leu Ala Val Asp
   65                  70                  75                  80
   Thr Phe Gly Arg Ile Asp Phe Ala His Asn Asn Ala Gly Ile Glu Gly
                   85                  90                  95
   Val Gln Ala Met Leu Ala Asp Tyr Pro Glu Glu Val Trp Asp Arg Val
               100                 105                 110
   Ile Glu Ile Asn Leu Lys Gly Val Trp Leu Cys Met Lys Tyr Glu Ile
           115                 120                 125
   Arg His Met Leu Lys Gln Gly Gly Gly Ala Ile Val Asn Thr Ser Ser
       130                 135                 140
   Val Ala Gly Leu Ala Gly Ser Arg Gly Val Ser Ala Tyr Val Ala Ser
   145                 150                 155                 160
   Lys His Gly Ile Val Gly Ile Thr Lys Ala Ala Ala Leu Glu Tyr Ala
                   165                 170                 175
   Arg Asn Gly Ile Arg Val Asn Ala Ile Cys Pro Gly Thr Ile His Thr
               180                 185                 190
   Ala Met Ile Asp Arg Phe Thr Gln Gly Asp Pro Gln Leu Leu Ala Gln
           195                 200                 205
   Phe Ala Glu Gly Glu Pro Ile Gly Arg Leu Gly Ser Pro Glu Glu Val
       210                 215                 220
   Ala Asn Ala Val Ile Trp Leu Cys Ser Asp Lys Ala Ser Phe Val Thr
   225                 230                 235                 240
   Gly Ala Thr Leu Ala Val Asp Gly Gly Arg Leu Ala
                   245                 250
<210> 2
   <211> 249
   <212> PRT
   <213> Rubrobacter xylanophilus DSM 9941
<400> 2
   Met Leu Glu Gly Lys Val Ala Val Ile Thr Gly Ala Gly Ser Gly Ile
   1               5                   10                  15
   Gly Arg Ala Thr Ala Leu Lys Phe Ala Arg Glu Gly Ala Arg Val Val
               20                  25                  30
   Ala Ala Glu Leu Asp Glu Arg Gly Gly Glu Gly Val Val Arg Glu Val
           35                  40                  45
   Arg Ser Leu Gly Gly Glu Ala Val Phe Val Arg Thr Asp Val Ser Glu
       50                  55                  60
   Phe Ala Gln Val Glu Asp Ala Val Glu Arg Ala Val Gly Glu Tyr Gly
   65                  70                  75                  80
   Thr Leu Asp Val Met Phe Asn Asn Ala Gly Ile Gly His Tyr Ala Pro
                   85                  90                  95
   Leu Leu Glu His Glu Pro Glu His Tyr Asp Arg Val Val Arg Val Asn
               100                 105                 110
   Gln Tyr Gly Val Tyr Tyr Gly Ile Leu Ala Ala Gly Arg Lys Met Val
           115                 120                 125
   Ala Leu Lys Asn Pro Gly Leu Ile Ile Asn Thr Ala Ser Val Tyr Ala
       130                 135                 140
   Phe Leu Ala Ser Pro Gly Val Ile Gly Tyr His Ala Ala Lys Gly Ala
   145                 150                 155                 160
   Val Lys Met Met Thr Gln Ala Ala Ala Leu Glu Leu Ala Pro His Gly
                   165                 170                 175
   Ile Arg Val Val Ala Ile Ala Pro Gly Gly Val Asp Thr Pro Ile Ile
   180 185 190
   Gln Gly Tyr Lys Asp Met Gly Leu Gly Glu Arg Leu Ala Arg Gly Gln
   195 200 205
   Met Arg Arg Arg Leu Gln Thr Pro Glu Gln Ile Ala Gly Ala Val Ala
   210 215 220
   Leu Leu Ala Thr Asp Glu Ala Asp Ala Ile Asn Gly Ser Val Val Met
   225 230 235 240
   Thr Asp Asp Gly Tyr Ala Glu Phe Lys
   245
<210> 3
   <211> 243
   <212> PRT
   <213> Candida magnoliae CBS 6396
<400> 3
   Met Ser Ala Thr Ser Asn Ala Leu Ile Thr Gly Ala Ser Arg Gly Met
   1               5                   10                  15
   Gly Glu Ala Thr Ala Ile Lys Leu Ala Leu Glu Gly Tyr Ser Val Thr
               20                  25                  30
   Leu Ala Ser Arg Gly Ile Glu Gln Leu Asn Ala Ile Lys Glu Lys Leu
           35                  40                  45
   Pro Ile Val Lys Lys Gly Gln Gln His Tyr Val Trp Gln Leu Asp Leu
       50                  55                  60
   Ser Asp Ile Glu Ala Ala Ser Thr Phe Lys Gly Ala Pro Leu Pro Ala
   65                  70                  75                  80
   Ser Ser Tyr Asp Val Phe Phe Ser Asn Ala Gly Val Val Asp Phe Ala
                   85                  90                  95
   Pro Phe Ala Asp Gln Ser Glu Thr Ala Gln Lys Asp Leu Phe Thr Val
               100                 105                 110
   Asn Leu Leu Ser Pro Val Ala Leu Thr Lys Thr Ile Val Lys Ala Ile
           115                 120                 125
   Ala Asp Lys Pro Arg Glu Thr Pro Ala His Ile Ile Phe Thr Ser Ser
       130                 135                 140
   Ile Val Gly Ile Arg Gly Val Pro Asn Val Ala Val Tyr Ser Ala Thr
   145                 150                 155                 160
   Lys Gly Ala Ile Asp Ser Phe Ala Arg Ser Leu Ala Arg Glu Phe Gly
                   165                 170                 175
   Pro Lys Asn Ile His Val Asn Cys Val Asn Pro Gly Thr Thr Arg Thr
               180                 185                 190
   Glu Met Thr Lys Gly Val Asp Leu Ala Ala Phe Gly Asp Val Pro Ile
           195                 200                 205
   Lys Gly Trp Ile Glu Val Asp Ala Ile Ala Asp Ala Val Leu Phe Leu
       210                 215                 220
   Ile Lys Ser Lys Asn Ile Thr Gly Gln Ser Leu Val Val Asp Asn Gly
   225                 230                 235                 240
   Phe Gly Val
<210> 4
   <211> 241
   <212> PRT
   <213> Candida magnoliae DSM 70638
<400> 4
   Met Thr Ser Thr Pro Asn Ala Leu Ile Thr Gly Gly Ser Arg Gly Ile
   1               5                   10                  15
   Gly Ala Ser Ala Ala Ile Lys Leu Ala Gln Glu Gly Tyr Ser Val Thr
               20                  25                  30
   Leu Ala Ser Arg Asp Leu Glu Lys Leu Thr Glu Val Lys Asp Lys Leu
           35                  40                  45
   Pro Ile Val Arg Gly Gly Gln Lys His Tyr Val Trp Gln Leu Asp Leu
       50                  55                  60
   Ala Asp Val Glu Ala Ala Ser Ser Phe Lys Ala Ala Pro Leu Pro Ala
   65                  70                  75                  80
   Ser Ser Tyr Asp Leu Phe Val Ser Asn Ala Gly Ile Ala Gln Phe Ser
                   85                  90                  95
   Pro Thr Ala Glu His Thr Asn Ser Glu Trp Leu Asn Ile Met Thr Ile
               100                 105                 110
   Asn Leu Val Ser Pro Ile Ala Leu Thr Lys Ala Leu Leu Gln Ala Val
           115                 120                 125
   Ser Gly Arg Ser Ser Glu Asn Pro Phe Gln Ile Val Phe Ile Ser Ser
       130                 135                 140
   Val Ala Ala Leu Arg Gly Val Ala Gln Thr Ala Val Tyr Ser Ala Ser
   145                 150                 155                 160
   Lys Ala Gly Thr Asp Gly Phe Ala Arg Ser Leu Ala Arg Glu Leu Gly
                   165                 170                 175
   Pro Gln Gly Val His Val Asn Val Val Asn Pro Gly Trp Thr Lys Thr
               180                 185                 190
   Asp Met Thr Glu Gly Val Glu Thr Pro Lys Asp Met Pro Ile Lys Gly
           195                 200                 205
   Trp Ile Gln Pro Glu Ala Ile Ala Asp Ala Val Val Phe Leu Ala Arg
       210                 215                 220
   Ser Lys Asn Ile Thr Gly Ala Asn Ile Val Val Asp Asn Gly Phe Ser
   225                 230                 235                 240
   Thr
<210> 5
   <211> 241
   <212> PRT
   <213> Candida magnoliae DSM 70638
<400> 5
   Met Thr Thr Thr Ser Asn Ala Leu Val Thr Gly Gly Ser Arg Gly Ile
   1               5                   10                  15
   Gly Ala Ala Ser Ala Ile Lys Leu Ala Gln Glu Gly Tyr Asn Val Thr
               20                  25                  30
   Leu Ala Ser Arg Ser Val Asp Lys Leu Asn Glu Val Lys Ala Lys Leu
           35                  40                  45
   Pro Ile Val Gln Asp Gly Gln Lys His Tyr Ile Trp Glu Leu Asp Leu
       50                  55                  60
   Ala Asp Val Glu Ala Ala Ser Ser Phe Lys Gly Ala Pro Leu Pro Ala
   65                  70                  75                  80
   Arg Ser Tyr Asp Val Phe Val Ser Asn Ala Gly Val Ala Ala Phe Ser
                   85                  90                  95
   Pro Thr Ala Asp His Asp Asp Lys Glu Trp Gln Asn Leu Leu Ala Val
               100                 105                 110
   Asn Leu Ser Ser Pro Ile Ala Leu Thr Lys Ala Leu Leu Lys Asp Val
           115                 120                 125
   Ser Glu Arg Pro Val Asp Lys Pro Leu Gln Ile Ile Tyr Ile Ser Ser
       130                 135                 140
   Val Ala Gly Leu His Gly Ala Ala Gln Val Ala Val Tyr Ser Ala Ser
   145                 150                 155                 160
   Lys Ala Gly Leu Asp Gly Phe Met Arg Ser Val Ala Arg Glu Val Gly
                   165                 170                 175
   Pro Lys Gly Ile His Val Asn Ser Ile Asn Pro Gly Tyr Thr Lys Thr
               180                 185                 190
   Glu Met Thr Ala Gly Ile Glu Ala Leu Pro Asp Leu Pro Ile Lys Gly
           195                 200                 205
   Trp Ile Glu Pro Glu Ala Ile Ala Asp Ala Val Leu Phe Leu Ala Lys
       210                 215                 220
   Ser Lys Asn Ile Thr Gly Thr Asn Ile Val Val Asp Asn Gly Leu Ile
   225                 230                 235                 240
   Ala
<210> 6
   <211> 759
   <212> DNA
   <213> Chloroflexus aurantiacus DSM 635
<400> 6
   atggagccac ctttcattgg gaaggttgcg ctggtcaccg gcgcagcagc cggtattggt    60
   cgtgcttcag cactggcgtt tgcccgtgag ggtgccaagg ttgtcgttgc tgatgtgaat   120
   gtcgagggcg gggaagagac gattgcgctg tgtcgggctt tgaataccga tgcaatgttc   180
   gtgcgttgtg atgtttcgca acgcgatgaa gtggagcgat taattgctct ggcagttgac   240
   acgttcggtc ggatcgactt tgcgcacaac aacgccggga ttgaaggcgt gcaggcaatg   300
   ctggccgatt atcccgaaga ggtctgggat cgggtgatcg agatcaacct caaaggggtc   360
   tggttgtgta tgaagtacga aatccggcac atgctcaagc agggtggcgg tgcgattgtg   420
   aatacctcat cggtcgccgg tctggccgga tcacgtggcg tttcggcgta tgtagccagc   480
   aagcacggta ttgttggtat taccaaagcg gcagcccttg agtatgcgcg taacggtatt   540
   cgtgtcaacg caatctgtcc aggtacgatt catactgcga tgatcgaccg ctttacccag   600
   ggtgatcccc aactgcttgc ccagttcgct gagggtgaac cgattggtcg gctcggctcg   660
   cctgaagagg tcgccaatgc ggtgatctgg ctctgctcag ataaggcttc gtttgtgacc   720
   ggagcgacac tggcggttga tggtggccgc ctggcgtaa                          759
<210> 7
   <211> 750
   <212> DNA
   <213> Rubrobacter xylanophilus DSM 9941
<400> 7
   atgctcgagg ggaaggtcgc ggtcatcacg ggggccggaa gcggcatagg ccgggccacc      60
   gcgctcaagt tcgcccgcga gggggcccgg gtcgtcgccg ccgagctcga cgagcgcggc 120
   ggggaggggg tggtccggga ggtgcgcagc ctcgggggcg aggcggtctt cgtccggacc 180
   gacgtctcgg agttcgcgca ggtggaggac gccgtcgagc gggcggtcgg ggagtacggc 240
   accctcgacg tgatgttcaa caacgccggc atcgggcact acgcccccct gctggagcac 300
   gagcccgagc actacgaccg ggtggtccgg gtgaaccagt acggcgtcta ctacgggata 360
   ctcgccgccg ggagaaagat ggtcgccctg aagaaccccg gcttgatcat caacaccgcc 420
   tcggtctacg ccttcctcgc ctcgccgggg gtcatcggct accacgccgc caagggggcg 480
   gtcaagatga tgacccaggc ggcggcgctg gagctcgccc cgcacggcat aagggtcgtc 540
   gccatcgccc cgggcggggt ggacaccccc atcatccagg gctacaagga catggggctc 600
   ggcgagaggc tggcccgcgg ccagatgcgc cgccggctcc agacccccga gcagatcgcc 660
   ggggcggtcg ccctgctcgc caccgacgag gccgacgcca taaacggctc ggtggtcatg 720
   accgacgacg gctacgcgga gttcaagtag 750
<210> 8
   <211> 732
   <212> DNA
   <213> Candida magnoliae CBS 6396
<400> 8
   atgtctgcta cttcgaacgc tcttatcact ggtgccagcc gcggaatggg cgaggccaca 60
   gctattaagc ttgcccttga ggggtacagc gtcacccttg catcacgcgg tattgagcag 120
   ctcaatgcca tcaaggaaaa actacccatc gtgaagaagg gccagcagca ctacgtttgg 180
   cagctcgatc ttagtgacat cgaggcggct tccaccttca agggggctcc tctgcctgcc 240
   agcagctacg acgtgttctt cagcaacgcc ggtgtggtgg actttgctcc gttcgcagac 300
   caaagcgaga ctgcgcaaaa ggacctgttc acggttaacc tgctgtcgcc tgttgcgttg 360
   accaagacca ttgttaaggc catcgccgac aagccccgcg agacgcctgc tcacattatc 420
   ttcacctcgt ccattgtcgg aattcgcggt gttcccaacg tggcggtcta cagcgccacc 480
   aagggcgcga ttgacagctt tgcgcgctcg cttgctcgtg agttcggtcc caagaacatc 540
   cacgttaact gcgtgaaccc gggcacgacg cgcaccgaga tgacaaaggg cgttgatctc 600
   gcggctttcg gcgatgttcc tatcaagggc tggatcgagg tcgatgcgat tgccgacgct 660
   gtgctgtttt tgatcaagtc caagaacatc actggccagt cgctcgttgt tgacaacgga 720
   ttcggtgttt aa 732
<210> 9
   <211> 726
   <212> DNA
   <213> Candida magnoliae DSM 70638
<400> 9
   atgacatcta cacctaatgc cctcatcacg ggaggcagcc gcggcattgg cgcttccgcc 60
   gccatcaaac tggctcaaga agggtacagc gtcacgctgg cgtcccgcga ccttgagaaa 120
   cttactgagg tcaaggacaa gctgccaatc gtgagaggtg gacagaaaca ctacgtttgg 180
   cagctcgatc ttgccgatgt ggaggctgca tcgtctttca aggcggctcc tctgccggcc 240
   agcagctacg atttgtttgt ttcgaacgcc ggaattgccc agttctcgcc tacggcagag 300
   catactaata gtgagtggct gaacattatg accattaact tagtgtcccc gattgccctg 360
   acgaaggctc ttttgcaggc cgtttctggg aggtcgagcg agaacccgtt tcagatcgtc 420
   ttcatctcgt cggttgcagc actacgtggc gttgcacaaa cggccgtcta cagtgcgtcg 480
   aaggctggta ctgatggatt cgcacgctca cttgctcgcg aactaggtcc tcaaggtgtt 540
   catgtgaacg tggtgaaccc tggctggact aagacagaca tgacggaagg agtcgaaacc 600
   ccaaaggaca tgcccattaa gggctggatc cagcctgagg caattgctga tgctgtagta 660
   ttccttgcga ggtcgaaaaa cattaccggc gcgaatattg tagtggacaa tggtttctcg 720
   acgtaa 726
<210> 10
   <211> 726
   <212> DNA
   <213> Candida magnoliae DSM 70638
<400> 10
   atgacgacta cttcaaacgc gcttgtcact ggaggcagcc gcggcattgg cgctgcctcc 60
   gccattaagc tggctcagga gggctacaat gttacgctgg cctctcgcag tgttgataaa 120
   ctgaatgaag taaaggcgaa actcccaatt gtacaggacg ggcagaagca ctacatttgg 180
   gaactcgatc tggctgatgt ggaagctgct tcgtcgttca agggtgctcc tttgcctgct 240
   cgcagctacg acgtctttgt ttcgaacgcg ggcgtcgctg cgttctcgcc cacagccgac 300
   cacgatgata aggagtggca gaacttgctt gccgtgaact tgtcgtcgcc cattgccctc 360
   acgaaggccc tcttgaagga tgtctccgaa aggcctgtgg acaagccact gcagattatc 420
   tacatttcgt cggtggccgg cttgcatggc gccgcgcagg tcgccgtgta cagtgcatct 480
   aaggccggtc ttgatggttt tatgcgctcc gtcgcccgtg aggtgggccc gaagggcatc 540
   catgtgaact ccatcaaccc cggatacacg aagactgaaa tgaccgcggg cattgaagcc 600
   cttcctgatt tgcctatcaa ggggtggatc gagcccgagg caattgctga cgcggttctg 660
   tttctggcaa agtccaagaa tatcaccggc acaaacattg tggtcgacaa tggcttgatt 720
   gcttaa 726
<210> 11
   <211> 38
   <212> DNA
   <213> Synthetic construct
<220>
   <221> misc_feature
   <222> (1)..(38)
<400> 11
   ggaattccat atgatggagc cacctttcat tgggaagg 38
<210> 12
   <211> 34
   <212> DNA
   <213> Synthetic construct
<220>
   <221> misc_feature
   <222> (1)..(34)
<400> 12
   cccaagctta ttattacgcc aggcggccac catc 34
<210> 13
   <211> 34
   <212> DNA
   <213> synthetic construct
<220>
   <221> misc_feature
   <222> (1)..(34)
<400> 13
   cccaagctta ttattacgcc aggcggccac catc 34
<210> 14
   <211> 35
   <212> DNA
   <213> synthetic construct
<220>
   <221> misc_feature
   <222> (1)..(35)
<400> 14
   ggaattccat atgatgtctg ctacttcgaa cgctc 35
<210> 15
   <211> 33
   <212> DNA
   <213> synthetic construct
<220>
   <221> mise_feature
   <222> (1)..(33)
<400> 15
   ccgctcgagt tattaaacac cgaatccgtt gtc 33
<210> 16
   <211> 35
   <212> DNA
   <213> synthetic construct
<400> 16
   cacatgcatg cagatgtctg ctacttcgaa cgctc 35
<210> 17
   <211> 34
   <212> DNA
   <213> synthetic construct
<220>
   <221> misc_feature
   <222> (1)..(34)
<400> 17
   gcccgagctc ttattaaaca ccgaatccgt tgtc 34
<210> 18
   <211> 12
   <212> PRT
   <213> Synthetic construct
<400> 18
   Asn Ala Leu Val Thr Gly Ala Ser Arg Gly Ile Gly
   1 5 10
<210> 19
   <211> 12
   <212> PRT
   <213> Synthetic construct
<400> 19
   Asn Ala Leu Val Thr Gly Gly Ser Arg Gly Ile Gly
   1 5 10
<210> 20
   <211> 12
   <212> PRT
   <213> Synthetic construct
<400> 20
   Asn Ala Leu Ile Thr Gly Gly Ser Arg Gly Ile Gly
   1 5 10
<210> 21
   <211> 12
   <212> PRT
   <213> Synthetic construct
<400> 21
   Asn Ala Leu Ile Thr Gly Ala Ser Arg Gly Ile Gly
   1 5 10
<210> 22
   <211> 12
   <212> PRT
   <213> Synthetic construct
<400> 22
   Asn Ala Leu Ile Thr Gly Gly Ser Arg Gly Met Gly
   1 5 10
<210> 23
   <211> 12
   <212> PRT
   <213> Synthetic construct
<400> 23
   His Ala Leu Val Thr Gly Ala Ser Arg Gly Ile Gly
   1 5 10
<210> 24
   <211> 7
   <212> PRT
   <213> Synthetic construct
<400> 24
   Gly Tyr Ser Val Thr Leu Ala
   1 5
<210> 25
   <211> 7
   <212> PRT
   <213> Synthetic construct
<400> 25
   Gly Tyr Asn Val Thr Leu Ala
   1 5
<210> 26
   <211> 7
   <212> PRT
   <213> Synthetic construct
<400> 26
   Gly Tyr Ser Val Thr Leu Val
   1 5
<210> 27
   <211> 7
   <212> PRT
   <213> Synthetic construct
<400> 27
   Gly Tyr Asn Val Thr Leu Val
   1 5
<210> 28
   <211> 8
   <212> PRT
   <213> Synthetic construct
<400> 28
   Phe Lys Gly Ala Pro Leu Pro Ala
   1 5
<210> 29
   <211> 8
   <212> PRT
   <213> Synthetic construct
<400> 29
   Phe Lys Ala Ala Pro Leu Pro Ala
   1 5
<210> 30
   <211> 6
   <212> PRT
   <213> Synthetic construct
<400> 30
   Phe Val Ser Asn Ala Gly
   1 5
<210> 31
   <211> 6
   <212> PRT
   <213> Synthetic construct
<400> 31
   Phe Phe Ser Asn Ala Gly
   1 5
<210> 32
   <211> 6
   <212> PRT
   <213> Synthetic construct
<400> 32
   Phe Val Cys Asn Ala Gly
   1 5
<210> 33
   <211> 6
   <212> PRT
   <213> Synthetic construct
<400> 33
   Phe Val Ala Asn Ala Gly
   1 5
<210> 34
   <211> 9
   <212> PRT
   <213> Synthetic construct
<400> 34
   Ser Pro Ile Ala Leu Thr Lys Ala Leu
   1 5
<210> 35
   <211> 9
   <212> PRT
   <213> Synthetic construct
<400> 35
   Ser Pro Val Ala Leu Thr Lys Thr Ile
   1 5
<210> 36
   <211> 9
   <212> PRT
   <213> Synthetic construct
<400> 36
   Ser Pro Ile Ala Leu Thr Lys Thr Leu
   1 5
<210> 37
   <211> 9
   <212> PRT
   <213> Synthetic construct
<400> 37
   Ser Pro Val Ala Met Thr Lys Ala Leu
   1 5
<210> 38
   <211> 9
   <212> PRT
   <213> Synthetic construct
<400> 38
   Ser Gln Ile Ala Leu Thr Lys Ala Leu
   1 5
<210> 39
   <211> 7
   <212> PRT
   <213> Synthetic construct
<400> 39
   Ala Val Tyr Ser Ala Ser Lys
   1 5
<210> 40
   <211> 7
   <212> PRT
   <213> Synthetic construct
<400> 40
   Ala Val Tyr Ser Ala Thr Lys
   1 5
<210> 41
   <211> 6
   <212> PRT
   <213> Synthetic construct
<400> 41
   Pro Ile Lys Gly Trp Ile
   1 5
<210> 42
   <211> 6
   <212> PRT
   <213> Synthetic construct
<400> 42
   Pro Ile Ser Gly Trp Ile
   1 5

## Beschreibung

Die vorliegende Erfindung betrifft ein Verfahren zur enantioselektiven enzymatischen Reduktion von Secodionderivaten der allgemeinen Formel I, wobei das Secodionderivat mit einer Oxidoreduktase/Dehydrogenase in Gegenwart von NADH oder NADPH als Cofactor reduziert wird.

Die industrielle Darstellung von Steroidhormonen erfolgt auf zwei, voneinander unabhängigen Wegen, nämlich zum einen ausgehend von natürlich vorkommenden Steroidverbindungen aus pflanzlichen Quellen und zum anderen totalsynthetisch durch enantioselektive Synthese aus prochiralen Vorstufen. Von diesen beiden Wegen gewinnt die Steroidtotalsynthese zunehmend an Bedeutung, zumal diese auch die Einführung von Strukturelementen erlaubt, die in natürlich vorkommenden Steroiden nicht enthalten sind.

Schlüsselbausteine der Totalsynthese von enantiomerenreinen Steroiden sind dabei Verbindungen der allgemeinen Formel I, welche auch als Secosteroide, 8,14-seco-gonatetraen-14,17-dione oder Secodione bezeichnet werden. Spezielle Vertreter dieser Gruppe sind zum Beispiel die Verbindungen Methylsecodion (Formel II, 13-Methyl-3-methoxy-8,14-seco-gona-1,3,5(10),9(11)-tetraen-14,17-dion) und Ethylsecodion (Formel III, 13-Ethyl-3-methoxy-8,14-seco-gona-1,3,5(10),9(11)-tetraen-14,17-dion), aus denen beispielsweise die pharmakologisch wirksamen Verbindungen Ethinylestradiol (Formel IV) und Norgestrel (Formel V) hergestellt werden können.

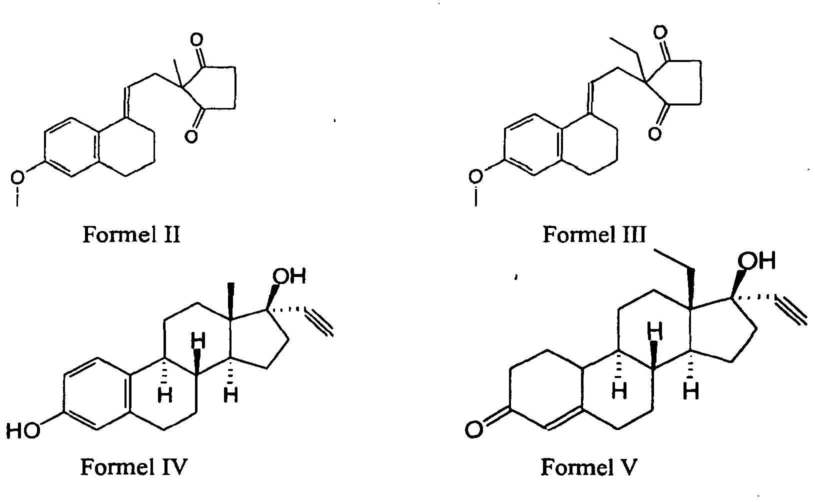

Schlüsselschritt bei der Darstellung enantiomerenreiner Steroidverbindungen ist dabei die Überführung der Verbindung der Formel I (z.B. II und III) in eine optisch aktive Verbindung mit vorgebildetem asymmetrischem C-13 durch enantioselektive Reduktion einer der Ketogruppen zur Hydroxygruppe. Die resultierenden optisch aktiven Hydroxysecosteroidverbindungen (Secole, Formeln VI bis IX) können anschließend durch Zyklisierung unter Erhalt der Chiralität zu chiralen Steroidverbindungen weiterverarbeitet werden.

Durch enantioselektive Reduktion einer Ketogruppe der Verbindung der Formel I können theoretisch vier optisch aktive Verbindungen gebildet werden (Formeln VI bis IX).

| | | | |
|---|---|---|---|
| 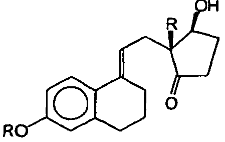 | 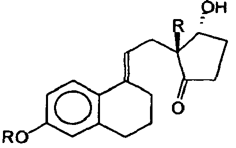 | 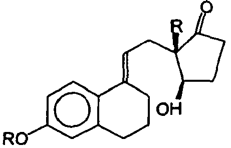 | 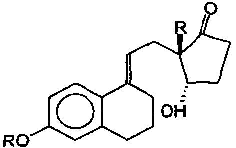 |
| Formel VI (17-beta-OH) | Formel VII (17-alpha-OH) | Formel VIII (14-beta-OH) | Formel IX (14-alpha-OH) |

Wirtschaftlich von besonderem Interesse sind dabei Verbindungen der Formel VI, bei denen die Hydroxygruppe an der Position 17 die beta-Konfiguration aufweist, da diese zu Derivaten des natürlichen Östron führen. Solche Verbindungen werden auch als 17-beta-Hydroxysecosteroide bezeichnet.

Die stereoselektive Reduktion von Secodionderivaten der allgemeinen Formel I mit Hilfe verschiedener Mikroorganismen wurde besonders intensiv in den 1960er und 1970er Jahren untersucht. Dabei konnte gezeigt werden, dass verschiedene Hefestämme der Gattung Candida, Debaryomyces, Kloeckera, Pichia, Cryptococcus, Rhodotorula, Torulopsis und Hansenula in der Lage sind Secodione zu den verschiedenen Hydroxyverbindungen zu reduzieren (US 3616226, US 1252524, US 3616225).

Insbesondere Hefen der Gattung Saccharomyces wie beispielsweise S. uvarum können vorteilhaft eingesetzt werden, um beispielsweise die entsprechenden 17-beta-Hydroxysecosteroide darzustellen (Kosmol et al; Liebigs Ann. Chem. 701,199 (1967)). Andere Hefestämme wie beispielsweise Saccharomyces drosophilarum reduzieren Secodion bevorzugt zum korrespondierenden 14-alpha-Hydroxysecosteroid (Acta microbiol. Acad. Sci. hung. 22,463-471 (1975)). Die Bildung des 14-alpha-Hydroxysecosteroids wird des weiteren auch durch Reduktion von Secodion mittels Bacillus thuringiensis beschrieben (Kosmol et al.; Liebigs Ann. Chem. 701,199 (1967)).

Gestagen- und Östrogenwirkstoffe finden weltweit breite Anwendung als Kontrazeptiva und in der Homonersatztherapie. Bis heute sind die meisten Synthesen von Östrogen- und Gestagenderivaten auf dem oben beschriebenen Reaktionsprinzip aufgebaut, dessen Schlüsselschritt die enantioselektive Reduktion von Secodionen zu den entsprechenden 17-beta-Hydroxysecosteroiden darstellt.

Die stereoselektive Reduktion der Secodionderivate wird dabei bis heute als Ganzzellbiotransformation mittels verschiedener Hefestämme der Gattung Pichia oder Saccharomyces durchgeführt. Diese Verfahren besitzen jedoch den Nachteil, dass nur sehr geringe Substratkonzentrationen von weit unter 1 % (i.d.R. 1 bis 5 g/l) realisierbar sind (US 3697379; Current Science, Feb.5 (1984), Vol 53. No. 3, Seite 124; Indian Journal of Experimental Biology, Vol.27, August 1989, Seite 742-743). Dadurch gestaltet sich insbesondere die Aufarbeitung und Isolierung des Reaktionsproduktes aus großen Volumina sowie die Abtrennung großer Mengen von Biomasse sehr aufwendig.

Szentirmai et al. (Acta Microbiologica Academiae Scientiarum Hungaricae, Bd. 22, Nr. 4, 1975, S. 463-470) beschreiben ein Verfahren zur enantioselektiven enzymatischen Reduktion von Secodionderivaten, wobei das Secodionderivat mit einer Oxidoreduktase/Dehydrogenase in Gegenwart von NADH oder NADPH als Cofaktor reduziert wird. Bei diesem Verfahren wird das Secodionderivat in einer Konzentration von lediglich 1 g/l im Reaktionsansatz eingesetzt.

Nach Wissen der Erfinder wurden die an der Reduktion beteiligten Enzyme bislang nicht isoliert, identifiziert und beschrieben. Ebenso wurden noch keine DNA-Sequenzen identifiziert, die für Oxidoreduktase codieren, mit denen die Reduktion von Secodionderivaten erreicht werden kann.

Die Erfindung stellt sich daher die Aufgabe, ein Verfahren bereitzustellen, mit dem Secodionderivate der allgemeinen Formel I, insbesondere solche der Formel II und III, enantioselektiv reduziert werden können. Unter anderem soll hierdurch auch die Herstellung der entsprechenden 17-beta-Hydroxysecosteroide möglich sein.

In einem ersten Aspekt wird diese Aufgabe erfindungsgemäß durch ein Verfahren zur enantioselektiven enzymatischen Reduktion von Secodionderivaten der allgemeinen Formel I gelöst, 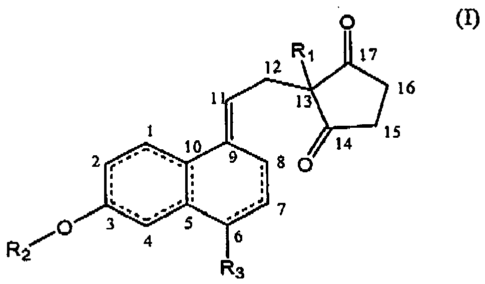 worin
R₁ Wasserstoff oder eine C₁-C₄-Alkylgruppe ist,
R₂ Wasserstoff, eine C₁-C₈-Alkylgruppe oder eine im Stand der Technik bekannte OH-Schutzgruppe, wie ein Ester, ist,
R₃ Wasserstoff, eine Methylgruppe oder ein Halogenid ist,
das Strukturelement 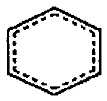 einen Benzolring oder einen C₆-Ring mit 0, 1 oder 2 C-C-Doppelbindungen repräsentiert, an den Positionen 6/7 oder 7/8 gegebenenfalls eine Doppelbindung enthalten ist und der Kohlenstoff an den Positionen 1, 2, 4, 5, 6, 7, 8, 9, 11, 12 und 16 unabhängig mit Wasserstoff, einer C₁-C₄-Alkylgruppe, einem Halogenid oder einer Phenylgruppe substituiert ist,
wobei das Secodionderivat mit einer Oxidoreduktase/Dehydrogenase in Gegenwart von NADH oder NADPH als Cofactor reduziert wird,
welches Verfahren dadurch gekennzeichnet ist, dass das Secodionderivat in einer Konzentration von ≥ 10 g/l im Reaktionsansatz eingesetzt wird und der durch die Oxidoreduktase/Dehydrogenase gebildete oxidierte Cofactor NAD oder NADP kontinuierlich regeneriert wird.

Dieses Verfahren stellt eine wesentliche Verbesserung der enantioselektiven enzymatischen Reduktion von Secodionderivaten gegenüber dem Stand der Technik dar. Das erfindungsgemäße Verfahren ermöglicht die Reduktion von Secodionderivaten zu den verschiedenen korrespondierenden Hydroxysecosteroiden mit freien Enzymen in Konzentrationsbereichen, die weit über die im Stand der Technik Beschriebenen hinausgehen.

Eine bevorzugte Ausführungsform des erfindungsgemäßen Verfahrens ist dadurch gekennzeichnet, dass die Oxidoreduktase/Dehydrogenase
a) eine Aminosäuresequenz aufweist, bei welcher mindestens 50% der Aminosäuren mit jenen der Aminosäuresequenz SEQ ID NO:1, SEQ ID NO:2, SEQ ID NO:3, SEQ ID NO:4 oder SEQ ID NO:5 identisch sind,
b) von der Nukleinsäuresequenz SEQ ID NO:6, SEQ ID NO:7, SEQ ID NO:8, SEQ ID NO:9 oder SEQ ID NO:10 codiert wird oder
c) von einer Nukleinsäuresequenz codiert wird, die mit SEQ ID NO:6, SEQ ID NO:7, SEQ ID NO:8, SEQ ID NO:9 oder SEQ ID NO:10 unter stringenten Bedingungen hybridisiert.

Von den Erfindern wurden Oxidoreduktasen identifiziert, die in der Lage sind, Secodionderivate zu Hydroxysecosteroiden zu reduzieren, und die rekombinant industriell hergestellt werden können. Durch das erfindungsgemäße Verfahren können wesentlich höhere Substratkonzentrationen erzielt werden als bei den aktuell verwendeten Ganzzellverfahren.

Im erfindungsgemäßen Verfahren kann die Oxidoreduktase mit der Sequenz SEQ ID NO:1, SEQ ID NO:2, SEQ ID NO:3, SEQ ID NO:4 oder SEQ ID NO:5 bzw. von diesen Polypeptiden ableitbares Polypeptid entweder vollständig gereinigt, teilweise gereinigt oder als Zellen, enthaltend das Polypeptid SEQ ID NO:1, SEQ ID NO:2, SEQ ID NO:3, SEQ ID NO:4 oder SEQ ID NO:5 eingesetzt werden. Die eingesetzten Zellen können dabei nativ, permeabilisiert oder lysiert vorliegen. Bevorzugt werden die Oxidoreduktasen bzw. davon ableitbare Derivate in einem geeigneten Wirtsorganismus, wie beispielsweise *Escherichia coli,* überexprimiert und das rekombinante Polypeptid zur Reduktion der Secodionderivate der allgemeinen Formel I eingesetzt.

Eine DNA-Sequenz SEQ ID NO:6, die für ein Polypeptid mit der SEQ ID NO:1 codiert, ist beispielsweise aus dem Genom des Organismus *Chloroflexus aurantiacus* DSM 635 erhältlich.

Eine DNA-Sequenz SEQ ID NO:7, die für ein Polypeptid mit der SEQ ID NO:2 codiert, ist beispielsweise aus dem Genom des Organismus *Rubrobacter xylanophilus* DSM 9941 erhältlich.

Eine DNA-Sequenz SEQ ID NO:8, die für ein Polypeptid mit der SEQ ID NO:3 codiert, ist erhältlich aus einer Hefe *Candida magnoliae* CBS 6396.

Oxidoreduktasen der SEQ ID NO:4 und SEQ ID NO:5 sind beispielsweise erhältlich durch Homologiescreening aus *Candida magnoliae* DSMZ 70638.

Unter einer Nukleinsäuresequenz, welche beispielsweise mit der SEQ ID NO:6 unter stringenten Bedingungen hybridisiert, versteht man ein Polynukleotid, welches mittels der Kolonie-Hybridisierungsmethode, der Plaque-Hybridisierungsmethode, der Southem-Hybridisierungsmethode oder Vergleichbarem unter Verwendung der SEQ ID NO:6 oder Teilsequenzen von SEQ ID NO:6 als DNA-Sonde identifiziert werden kann. Zu diesem Zweck wird das an einem Filter immobilisierte Polynukleotid beispielsweise mit der SEQ ID NO:6 in einer 0,7-1 M NaCl-Lösung bei 60°C hybridisiert. Die Hybridisierung wird, wie z.B. in Molecular Cloning, A Laboratory Manual, Second Edition (Cold Spring Harbor Laboratory Press, 1989) oder ähnlichen Publikationen beschrieben, durchgeführt. Anschließend wird der Filter mit 0,1 bis 2-facher SSC-Lösung bei 65°C gewaschen, wobei unter 1-facher SSC-Lösung ein Gemisch, bestehend aus 150 mM NaCl und 15 mM Natriumcitrat, verstanden wird.

Ein Polynukleotid, das mit den Polynukleotiden SEQ ID NO:6, SEQ ID NO:7, SEQ ID NO:8, SEQ ID NO:9 oder SEQ ID NO:10 aus der Sequenzliste unter oben genannten stringenten Bedingungen hybridisiert, sollte zumindest 60% Sequenzidentität mit den Polynukleotidsequenzen SEQ ID NO:6, SEQ ID NO:7, SEQ ID NO:8, SEQ ID NO:9 oder SEQ ID NO:10, besser zumindest 80% Identität, noch besser 95% Identität aufweisen.

Eine weitere bevorzugte Ansführungsform des erfindungsgemäßen Verfahrens ist dadurch gekennzeichnet, dass die Oxidoreduktase/Dehydrogenase eine Länge von 230 bis 260 Aminosäuren aufweist und eine oder mehrere der Partialsequenzen, ausgewählt aus der Gruppe bestehend aus [den Sequenzen SEQ ID NO:18 bis SEQ ID NO:42] umfasst.

In den erfindungsgemäßen Verfahren wird als Co-Faktor NADH oder NADPH eingesetzt. Unter dem Begriff "NADP" wird Nicotinamid-adenin-dinucleotid-phosphat, unter "NADPH" reduziertes Nicotinamid-adenin-dinucleotid-phosphat verstanden. Der Begriff "NAD" bedeutet Nicotinamid-adenin-dinucleotid, der Begriff "NADH" reduziertes Nicotinamid-adenin-dinucleotid.

Gemäß einer bevorzugten Ausführungsform des erfindungsgemäßen Verfahrenswird der oxidierte Cofactor NAD oder NADP durch Oxidation eines Alkohols regeneriert.

Als Cosubstrat werden dabei bevorzugt primäre und sekundäre Alkohole, wie Methanol, 2-Propanol, 2-Butanol, 2-Pentanol, 3-Pentanol, 4-Methyl-2-pentanol, 2-Hexanol, 2-Heptanol, 2-Octanol oder Cyclohexanol eingesetzt. Der Anteil des Cosubstrates für die Regenerierung kann 5 bis 95 Vol%, bezogen auf das Gesamtvolumen, betragen.

Vorzugsweise wird zur Cofaktorregenerierung ein sekundärer Alkohol mit der allgemeinen Formel RₓR_{y}CHOH verwendet wird, wobei Rₓ und R_{y} unabhängig voneinander Wasserstoff, eine verzweigte oder unverzweigte C₁-C₈-Alkylgruppe sind und C_{insgesamt} ≥ 3.

Gemäß einer anderen bevorzugten Ausführungsform der erfindungsgemäßen Verfahren wird zur Regenerierung des Cofaktors zusätzlich eine Oxidoreduktase/ Dehydrogenase zugesetzt.

Geeignete NADH-abhängige Alkoholdehydrogenasen sind beispielsweise erhältlich aus Bäckerhefe, aus *Candida parapsilosis* (CPCR) (US 5,523,223 und US 5,763,236, Enzyme Microb. Technol., 1993, 15(11):950-8), *Pichia capsulata* (DE 10327454.4), aus *Rhodococcus erythropolis* (RECR) (US 5,523,223), *Norcardia fusca* (Biosci. Biotechnol. Biochem., 63(10), 1999, S. 1721-1729; Appl. Microbiol. Biotechnol, 2003, 62(4):380-6; Epub 2003, Apr. 26) oder *Rhodococcus ruber* (J. Org. Chem., 2003, 68(2):402-6). Geeignete Cosubstrate für diese Alkoholdehydrogenasen sind beispielsweise die bereits genannten sekundären Alkohole wie 2-Propanol (Isopropanol), 2-Butanol, 2-Pentanol, 4-Methyl-2-pentanol, 2-Octanol oder Cyclohexanol.

Geeignete sekundäre Alkoholdehydrogenasen zur Regenerierung des NADPH sind beispielsweise solche, wie beschrieben und isoliert aus Organismen der Ordnung Lactobacillales, z.B. *Lactobacillus kefir* (US 5,200,335), *Lactobacillus brevis* (DE 19610984 A1; Acta Crystallogr. D. Biol. Crystallogr. 2000 Dec; 56 Pt 12:1696-8), *Lactobacillus minor* (DE 10119274), *Leuconostoc carnosum* (A 1261/2005, Kl. C12N) oder solche, wie beschrieben aus *Thermoanerobium brockii*, *Thermoanerobium ethanolicus* oder *Clostridium beijerinckii*.

Zur Cofactorregenerierung können prinzipiell aber auch andere enzymatische Systeme verwendet werden. Beispielsweise kann die Cofactorregenerierung mittels NAD- oder NADP-abhängiger Formiat-Dehydrogenase (Tishkov et al., J. Biotechnol Bioeng. [1999] 64, 187-193, Pilot-scale production and isolation of recombinant NAD and NADP specific formate dehydrogenase) durchgeführt werden. Geeignete Cosubstrate der Formiat-Dehydrogenase sind beispielsweise Salze der Ameisensäure, wie Ammoniumformiat, Natriumformiat oder Calciumformiat.

Der in den erfindungsgemäßen Verfahren erreichte TTN (total turn over number = mol reduzierte Secodionverbindung / mol eingesetzter Cofactor) liegt in der Regel im Bereich von 10² bis 10⁵, vorzugsweise beträgt er jedoch ≥ 10³.

Gemäß einer bevorzugten Ausführungsform werden die erfindungsgemäßen Verfahren in einem wässrigen organischen Zweiphasensystem durchgeführt.

Demgemäß erfolgt die Umsetzung des Secodionderivates in einem Zweiphasensystem, enthaltend beispielsweise einen 2-Alkohol zur Cofactorregenerierung, eine Oxidoreduktase , Wasser, Cofaktor und die Secodionverbindung. Es können aber noch zusätzliche organische Lösungsmittel enthalten sein, die nicht an der Cofactorregenerierung beteiligt sind, d.h. keine oxidierbare Hydroxygruppen enthalten. Vorzugsweise werden als zusätzliche organische Lösungsmittel Diethylether, tertiär-Butylmethylether, Diisopropylether, Dibutylether, Ethylacetat, Butylacetat, Heptan, Hexan, Toluol, Dichlormethan, Cyclohexan oder Gemische davon eingesetzt.

Der Anteil der nicht wassermischbaren organischen Komponenten des Zweiphasensystems kann dabei von 10% bis 90%, bevorzugt von 20% bis 80%, bezogen auf das Gesamtvolumen des Reaktionsansatzes, betragen. Der wässrige Anteil kann von 90% bis 10%, bevorzugt von 80% bis 20%, bezogen auf das Gesamtvolumen des Reaktionsansatzes, betragen.

Dem Wasser kann auch ein Puffer zugesetzt werden, beispielsweise Kaliumphosphat-, Tris/HCl-, Glycin- oder Triethanolamin-Puffer mit einem pH-Wert von 5 bis 10, vorzugsweise von 6 bis 9. Der Puffer kann zusätzlich noch Ionen zur Stabilisierung oder Aktivierung beider Enzyme enthalten, beispielsweise Magnesiumionen oder Zinkionen.

Des weiteren können in den erfindungsgemäßen Verfahren noch weitere Zusätze zur Stabilisierung der verwendeten Enzyme eingesetzt werden, beispielsweise Glycerin, Sorbitol, 1,4-DL-Dithiothreit (DTT) oder Dimethylsulfoxid (DMSO).

Die Konzentration des Cofaktors NAD(P)H, bezogen auf die wässrige Phase, beträgt von 0,001 mM bis 10 mM, insbesondere von 0,01 mM bis 1,0 mM. Die Temperatur kann in Abhängigkeit von den speziellen Eigenschaften der eingesetzten Enzyme von 10°C bis 70°C betragen, bevorzugt von 20°C bis 35°C.

Die zu reduzierenden Secodionderivate sind in der Regel schwer wasserlöslich. Das Substrat kann daher während der Reaktion vollständig oder auch unvollständig gelöst vorliegen. Ist das Substrat im Reaktionsgemisch nicht vollständig gelöst, so liegt ein Teil des Substrates in fester Form vor und kann so eine dritte feste Phase bilden. Das Reaktionsgemisch kann während der Umsetzung auch zeitweise eine Emulsion bilden.

Das Secodionderivat der allgemeinen Formel I wird in den erfindungsgemäßen Verfahren vorzugsweise in einer Menge von 10g/l bis 500 g/l, bevorzugt von 25 g/l bis 300 g/l, besonders bevorzugt von 50g/l bis 200g/l, bezogen auf das Gesamtvolumen, im Reaktionsansatz eingesetzt.

Bevorzugte Ausführungsformen der Erfindung sind ferner dadurch gekennzeichnet, dass als Secodionderivat 13-Ethyl-3-methoxy₋8,14-seco-gona-1,3,5(10),9(11)-tetraen-14,17-dion (Ethylsecodion - Formel III) oder 13-Methyl-3-methoxy-8,14-seco-gona-1,3,5(10),9(11)-tetraen-14,17-dion (Methylsecodion - Formel II) eingesetzt wird.

Die erfindungsgemäßen Verfahren werden beispielsweise in einem Reaktionsgefäß aus Glas oder Metall durchgeführt. Dazu werden die Komponenten einzeln in das Reaktionsgefäß überführt und unter einer Atmosphäre von beispielsweise Stickstoff oder Luft gerührt. Je nach eingesetzter Secodionverbindung und Oxidoreduktase beträgt die Reaktionszeit von einer Stunde bis 7 Tage, insbesondere von 2 Stunden bis 48 Stunden. In dieser Zeit wird die Secodionverbindung zu mindestens 50 % zur korrespondierenden Hydroxysecosteroidverbindung reduziert.

Die vorliegende Erfindung wird nachfolgend anhand von Beispielen näher erläutert.

### Beispiel 1, nicht erfindungsgemäß

### Klonierung einer Oxidoreduktase aus Chloroflexus auratiacus DSM 635

### A) Anzucht von Chloroflexus auratiacus DSM 635

Zellen von *Chloroflexus auratiacus DSM 635* wurden in folgendem Medium (pH 8,2) bei 48°C in einem Bakterien-Inkubator bei Licht kultiviert: 0,1 % Hefeextrakt, 0,1 % Glycyl-Glycin, 0,01 % Na₂HPO₄ x 2 H₂O, 0,01 % MgSO₄ x 7 H₂O 0,01 % KNO₃, 0,05 % NaNO₃, 0,01 % NaCl, 0,005 % CaCl₂ x 2 H₂O, 5 ml einer 0,01 % Fe(III)citrat-Lösung, 1 ml Spurenelemente-Lösung SL-6 [500 µl/l H₂SO₄, 2,28 g/l MnSO₄ x H₂O 500 mg/l ZnSO₄ x 7 H₂O 500 mg H₃BO₃, 25 mg/l CuSO₄ x 5 H₂O, 25 mg/l Na₂MoO₄ x 2 H₂O, 45 mg/l CoCl₂ x 6 H₂O] Am Tag 12 der Kultivierung wurden Zellen mittels Zentrifugation vom Kulturmedium separiert und bei -80°C gelagert.

### B) Amplifikation des für selektive Oxidoreduktase kodierenden Gens

Genomische DNA wurde nach der in "Molecular cloning" von Manniatis & Sambrook beschriebenen Methode extrahiert. Die resultierende Nukleinsäure diente als Matrize für die Polymerase-Kettenreaktion (PCR) mit spezifischen Primern, die von der in der NCBI Datenbank unter der Nummer 76258197 publizierten Gen-Sequenz abgeleitet wurden. Dabei wurden die Primer für eine nachfolgende Klonierung in einen Expressionsvektor 5'-terminal mit Restriktionsschnittstellen für die Endonukleasen Nde I und Hind III bzw. Sph I versehen (SEQ ID NO: 11, SEQ ID NO:12, SEQ ID NO: 13).

Die Amplifizierung wurde in einem PCR-Puffer [10 mM Tris-HCl, (pH 8,0); 50 mM KCl; 10 mM MgSO₄; 1 mM dNTP Mix; je 20 pMol Primer und 2,5 U Platinum Pfx DNA-Polymerase (Invitrogen)] mit 500 ng genomischer DNA und folgenden Temperatur-Zyklen durchgeführt:
Zyklus 1: 94°C, 2 min
Zyklus 2 x 30: 94°C, 30 sec
   56°C, 30 sec
   68°C, 60 sec
Zyklus 3: 68°C, 7 min
   4°C, ∞

Das resultierende PCR-Produkt einer Größe von etwa 750 bp wurde nach der Reinigung über ein 1 % Agarose-Gel mit Hilfe von Endonukleasen *Nde I* und *Hind III,* bzw. mit Endonucleasen *Sph I* und *Hind III* restringiert und in das mit gleichen Endonukleasen behandelte Rückgrad des pET21a Vectors (Novagen) bzw. pQE70 Vectors (Qiagen) ligiert. Nach der Transformation von 2 µl des Ligationsansatzes in *E.coli* Top 10 F' Zellen (Invitrogen) wurden Plasmid-DNAs Ampicillin-(bzw. Kanamycin)-resistenter Kolonien mittels einer Restriktionsanalyse mit den Endonukleasen *Nde I und Hind III* bzw. den Endonucleasen *Sph I* und *Hind III* auf das Vorhandensein eines 750 bp großen Inserts getestet. Plasmid-Präparationen aus den für das Fragment positiven Klonen wurden einer Sequenzanalyse unterzogen und anschließend in *Escherichia coli* BL21 Star (Invitrogen), bzw *E.coli* RB791 (genetic stock, Yale) transformiert.

### Beispiel 2, nicht erfindungsgemäß

### Expression von rekombinanter Chloroflexus Oxidoreduktase in E.coli

Die mit dem Expressionskonstrukt transformierten *Escherichia coli*-Stämme BL21 Star (Invitrogen, Karlsruhe, Deutschland) bzw. RB791 (E.coli genetic stock, Yale, USA) wurden in 200 ml LB-Medium (1% Tryptone, 0,5% Hefeextrakt, 1 % NaCl) mit Ampicillin (50 µg/ml) bzw. Carbenicillin (50 µg/ml) kultiviert, bis eine optische Dichte (OD) von 0,5, gemessen bei 550 nm, erreicht wurde. Die Expression von rekombinantem Protein wurde durch Zugabe von Isopropylthiogalaktosid (IPTG) in einer Konzentration von 0,1 mM induziert. Nach 8 Stunden bzw. 16 Stunden Induktion bei 25°C und 220 Upm wurden die Zellen geerntet und bei -20°C eingefroren. Für den Aktivitätstest wurden 10 mg Zellen mit 500 µl 100 mM TEA Puffer pH 7,0 und 500 µl Glasperlen versetzt und 10 min mittels einer Kugelmühle aufgeschlossen. Das erhaltene Lysat wurde dann verdünnt für die entsprechenden Messungen eingesetzt. Der Aktivitätstest setzte sich wie folgt zusammen: 870 µl 100 mM TEA Puffer pH 7,0, 160 µg NADH, 10 µl verdünntes Zelllysat. Die Reaktion wurde durch Zugabe von 100 µl einer 100 mM Substratlösung zum Reaktionsgemisch gestartet.

Für die Enzymgewinnung in großen Mengen wurden 30 g Zellen in 150 ml Triethanolaminpuffer (100 mM, pH 7, 2 mM MgCl₂, 10 % Gylcerin) resuspendiert und mittels Hochdruckhomogenisator aufgeschlossen. Die Enzymlösung wurde anschließend mit 150 ml Glycerin versetzt und bei -20°C gelagert.

### Beispiel 3, nicht erfindungsgemäß

### Anzucht von Organismen und Screening nach reduktiver Unsetzung von Ethylsecodion (Formel III)

Zum Screening wurden die Hefestämme *Pichia farinosa* DSM 70362, *Candida gropengiesseri* MUCL 29836, *Candida vaccinii* CBS 7318, *Pichia farinosa* DSM 3316, *Saccharomyces cerevisiae* CBS 1508 und *Candida magnoliae* CBS 6396 in folgendem Medium kultiviert: Hefeextrakt (5), Pepton (5) und Glucose (20) (Zahlen in Klammern sind jeweils g/l). Das Medium wurde bei 121°C sterilisiert und die Hefen wurden ohne weitere pH-Regulierung bei 25°C auf einem Schüttler bei 140 Umdrehungen pro Minute kultiviert.

Die reduktive Umsetzung von Ethylsecodion der Formel III zur entsprechenden Hydroxysecosteroidverbindung wurde in folgenden Ganzzellbiotransformations-Ansätzen getestet:
400 mg frisch geerntete Zellen wurden in einem Ansatz mit 50 mg Glucose, 10 mg Ethylsecodion der Formel III und 900 µl 100 mM Trieethanolamin Puffer (TEA) pH 7,0 für 24 Stunden bei 28°C und 1400 Upm geschüttelt. Anschließend wurden die Ansätze mit 1 ml Dichlormethan extrahiert, zentrifugiert, mit Stickstoff getrocknet und in Acetonitril aufgenommen zur HPLC-Analyse gegeben.

Die Screeningergebnisse sind in Tabelle 1 zusammengefasst.

**Tabelle 1**

| Stamm Nr. | Mikrorganismus | Umsetzung von Ethylsecodion nach 24 Stunden mit Wt Stämmen |
|---|---|---|
| | | Ansatz 24 h |
| DSM 70362 | Pichia farinosa | 0,7 % |
| MUCL 29836 | Candida gropengiesseri | 0,2 % |
| CBS 7318 | Candida vaccinii | 3,2 % |
| DSM 3316 | Pichia farinosa | 15,8 % |
| CBS 1508 | Saccharomyces cerevisiae | 0, 7% |
| CBS 6396 | Candida magnoliae | 41 % |

Der Stamm CBS 6396 zeigte die höchste Umsetzung von Ethylsecodion und wurde daher als Ausgangsorganismus für die Herstellung einer cDNA Bibliothek ausgesucht.

### Beispiel 4: nicht erfindungsgemäß

### Herstellung einer cDNA Bibliothek aus Candida magnoliae CBS 6396 und Klonierung von Oxidoreduktase

### A) Isolierung (gesamt und mRNA) sowie Herstellung der cDNA Bibliothek

600 mg frische Zellen wurden in 2,5 ml eiskaltem LETS-Puffer resuspendiert. Zu dieser Zellsuspension wurden 5 ml (etwa 20 g) in Salpetersäure gewaschener Glasperlen, equilibriert mit 3 ml Phenol (pH 7,0), hinzugegeben. Der gesamte Ansatz wurde dann abwechselnd jeweils 30 sec Vortex, 30 sec Kühlen auf Eis insgesamt 10 min behandelt. Anschließend wurden 5 ml eiskalter LETS-Puffer hinzugegeben und nochmals kräftig mit Vortex gemischt. Diese Zellsuspension wurde für 5 min bei 11000 g bei 4°C zentrifugiert. Die wässrige Phase wurde gewonnen und mit dem gleichen Volumen an Phenol:Chloroform:Isoamylalkohol (24:24:1) zweimal extrahiert. Anschließend folgte die Extraktion mit Chloroform. Nach der letzten Extraktion wurde die Gesamt-RNA durch Zugabe von 1/10 Vol an 5 M LiCl₂ bei -20°C 4 h präzipitiert.

1 mg so gewonnene Gesamt-RNA wurde über Oligo-dT Cellulose (NEB Biolabs) für die Anreicherung der mRNA-Moleküle benutzt. Nach der anschließenden Präzipitation wurden 5 µg mRNA für die cDNA Synthese (pBluescript IIXR cDNA Library Construction kit, Stratagene) verwendet. Die nach den Angaben des Herstellers konstruierte Bibliothek wurde in XL-10 Gold *E.coli* transformiert und auf die Aktivität einer ADH gescreent. Anhand der Extinktionsabnahme mit NADPH bzw. NADH als Cofaktor und Ethylsecodion (Formel III) als Substrat wurde ein Klon (cM4) identifiziert und isoliert. Die Sequenzierung des aus dem Klon isolierten Plasmids mit Primer T7 und Primer T3 resultierte in einer ORF von 789 bp. Dieses Fragment codierte für ein Fusionsprotein einer Größe von 262 Aminosäuren und bestand aus dem a-Fragment der ß-Galactosidase und der Sequenz einer putativen short-chain Alkoholdehydrogenase.

### B) Synthese eines für eine short-chain ADH aus Candida magnoliae CBS 6396 kodierenden Volllänge-Transkripts durch PCR

Es wurden spezifische Primer für eine nachfolgende Klonierung des Volllänge-Transkripts in die passenden Expressionssysteme konstruiert. Dabei wurde 5'-Primer mit einer Erkennungssequenz für *Nde I bzw. Sph I* und 3'-Primer mit einer Erkennungssequenz für *XhoI bzw. SacI* modifiziert (SEQ ID NO: 14, SEQ ID NO: 15, SEQ ID NO:16, SEQ ID NO:17). Plasmid-DNA, isoliert aus dem Klon (cM4) der Expressionsbibliothek von *Candida magnoliae*, diente als Matrize für die Polymerase-Kettenreaktion. Die Amplifizierung wurde in einem PCR-Puffer [10 mM Tris-HCl (pH 8,0); 50 mM KCl; 10 mM MgSO₄; 1 mM dNTP Mix; je 20 pMol Primer und 2,5 U Platinum Pfx DNA-Polymerase (Invitrogen)] mit 50 ng Matrize und folgenden Temperatur-Zyklen durchgeführt:
Zyklus 1: 94°C, 2 min
Zyklus 2 x 30:94°C, 15 sec
   58°C, 30 sec
   68°C, 75 sec
Zyklus 3: 68°C, 7 min
   4°C, ∞

Das resultierende PCR-Produkt wurde nach der Reinigung über ein 1 % Agarose-Gel mit Hilfe der Endonukleasen *Nde I* und *Xho I bzw.* der Endonucleasen *Sph I und Sac I* restringiert und in das mit gleichen Endonukleasen behandelte Rückgrad des pET21a a Vectors (Novagen) bzw. pQME70 Vectors ligiert. Nach der Transformation von 2 µl des Ligationsansatzes in *E.coli* Top 10 F' Zellen (Invitrogen) wurden Plasmid-DNAs Ampicillin-(bzw. Kanamycin)-resistenter Kolonien mittels einer Restriktionsanalyse mit den Endonukleasen *Nde I* und *XhoI* bzw. den Endonucleasen *Sph I* und *SacI* auf das Vorhandensein eines 750 bp großen Inserts getestet. Die Expressionskonstrukte pET21-MgIV und pQME70-MgIV wurden sequenziert. Das für eine short-chain Oxidoreduktase codierende Gen aus *Candida magnoliae* besaß einen offene Leserahmen von insgesamt 729 bp (in SEQ ID NO:8 enthalten), das einem Protein von 243 Aminosäuren ensprach (SEQ ID NO:3)

### Beispiel 5, nicht erfindungsgemäß

### Expression rekombinanter Oxidoreduktase in E.coli Zellen

Kompetente *Escherichia coli* StarBL21(De3)-Zellen (Invitrogen) bzw. RB791-Zellen (E.coli genetic stock, Yale, USA) wurden mit den für die Oxidoreduktase codierenden Expressionskonstrukten pET21-MgIV bzw. pQME70-MgIV transformiert. Die mit dem Expressionskonstrukten transformierten *Escherichia* coli-Kolonien wurden dann in 200 ml LB-Medium (1% Trypton, 0,5% Hefeextrakt, 1 % NaCl) mit 50 µg/ml Ampicillin bzw. 40 µg/ml Kanamycin kultiviert, bis eine optische Dichte von 0,5, gemessen bei 550 nm, erreicht wurde. Die Expression von rekombinantem Protein wurde durch Zugabe von Isopropylthiogalaktosid (IPTG) in einer Konzentration von 0,1 mM induziert. Nach 16 Stunden Induktion bei 25°C und 220 Upm wurden die Zellen geerntet und bei -20°C eingefroren. Für den Aktivitätstest wurden 10 mg Zellen mit 500 µl 100 mM TEA Puffer pH 7,0, 1 mM MgCl₂ und 500 µl Glasperlen versetzt und 10 min mittels einer Kugelmühle aufgeschlossen. Das erhaltene Lysat wurde dann verdünnt für die entsprechenden Messungen eingesetzt.

Der Aktivitätstest setzte sich wie folgt zusammen: 960 µl 100 mM TEA Puffer pH 7,0, 1bmM MgCl₂, 160 µg NADPH, 10 µl verdünntes Zelllysat. Die Reaktion wurde durch Zugabe von 10 µl einer 100 mM Substratlösung in 70 % Methanol zum Reaktionsgemisch gestartet.

Für die Enzymgewinnung in großen Mengen wurden 30 g Zellen in 150 ml Triethanolaminpuffer (100 mM, pH 7, 2 mM MgCl₂, 10 % Gylcerin) resuspendiert und mittels Hochdruckhomogenisator aufgeschlossen. Die Enzymlösung wurde anschließend mit 150 ml Glycerin versetzt und bei -20°C gelagert.

### Beispiel 6

### Reduktion von Ethylsecodion (Formel III) mittels Oxidoreduktase SEQ ID NO:1

Für die Reduktion von Ethylsecodion (Formel III) wurde in einem Reaktionsgefäß ein Gemisch aus 800 µl Puffer (100 mM Kaliumphosphat, pH = 7, 2 mM MgCl₂), 1,2 ml 2-Propanol, 0,08 mg NAD, 100 mg Ethylsecodion (Formel III) und 1 ml Enzymsuspension Oxidoreduktase SEQ ID NO:1 (siehe Beispiel 3) für 24 h bei Raumtemperatur unter ständiger Durchmischung inkubiert. Nach 96 h waren >90 % des eingesetzten Ethylsecodions (Formel III) reduziert.

Nach Beendigung der Reaktion wurde das Reaktionsgemisch durch Extraktion mit Dichlormethan aufgearbeitet, die das Produkt enthaltende organische Phase wurde abgetrennt und die 17-beta-Hydroxyverbindung (Ethylsecol) durch Abdampfen/Abdestilieren des Lösungsmittels gewonnen.

Die Umsetzung des Ethylsecodions zum Ethylsecol wurde mittels HPLC verfolgt. Hierzu wurde eine Trennsäule EC 125/4 Nucleodur 100-5 C18ec (Machery-Nagel, Düren, Deutschland) mit Acetonitril und Wasser als Laufmittel verwendet. Zur Analytik wurde ein linearer Gradient des Acetonitrilanteils im Laufmittel von 30 % auf 70 % angewendet. Die Identifizierung der Reaktionsprodukte erfolgte durch Vergleich mit Referenzsubstanzen.

### Beispiel 7

### Reduktion Von Ethylsecodion (Formel III) mittels Oxidoreduktase SEO ID NO:2

Für die Reduktion von Ethylsecodion (Formel III) wurde in einem Reaktionsgefäß ein Gemisch aus 250 µl Puffer (100 mM Triethanolamin, pH = 8, 2 mM MgCl₂), 250 µl 4-Methyl-2-pentanol, 0,02 mg NAD, 25 mg Ethylsecodion (Formel III) und 25 µl Enzymsuspension Oxidoreduktase SEQ ID NO:2 (siehe Beispiel 3) für 96 h bei Raumtemperatur unter ständiger Durchmischung inkubiert. Nach 96 h waren >30 % des eingesetzten Ethylsecodions (Formel III) zur Hydroxyverbindung reduziert.

Nach Beendigung der Reaktion wurde das Reaktionsgemisch durch Extraktion mit Dichlormethan aufgearbeitet, die das Produkt enthaltende organische Phase wurde abgetrennt und die 17-beta-Hydroxyverbindung (Ethylsecol) durch

Abdampfen/Abdestilieren des Lösungsmittels gewonnen.

### Beispiel 8

### Reduktion von Ethylsecodion (Formel III) mittels Oxidoreduktase SEQ ID NO:3

Für die Reduktion von Ethylsecodion (Formel III) wurde in einem Reaktionsgefäß ein Gemisch aus 100 µl Puffer (100 mM Triethanolamin, pH = 7, 2 mM MgCl₂), 400 µl 4-Methyl-2-pentanol, 0,02 mg NADP, 25 mg Ethylsecodion (Formel III) und 100 µl Enzymsuspension Oxidoreduktase SEQ ID NO:3 (siehe Beispiel 3) für 72 h bei Raumtemperatur unter ständiger Durchmischung inkubiert. Nach 72 h waren >95 % des eingesetzten Ethylsecodions (Formel III) zur Hydroxyverbindung reduziert.

### Beispiel 9

### Reduktion von Ethylsecodion (Formel III) mittels Oxidoreduktase SEO ID NO:4

Für die Reduktion von Ethylsecodion (Formel III) wurde in einem Reaktionsgefäß ein Gemisch aus 200 µl Puffer (100 mM Triethanolamin, pH = 9, 2 mM MgCl₂), 300 µl 2-Heptanol, 0,025 mg NADP, 100 mg Ethylsecodion (Formel III) und 50 µl Enzymsuspension Oxidoreduktase SEQ ID NO:4 (siehe Beispiel 3) für 72 h bei Raumtemperatur unter ständiger Durchmischung inkubiert. Nach 72 h waren >80 % des eingesetzten Ethylsecodions (Formel III) zur Hydroxyverbindung reduziert.

### Beispiel 10

### Reduktion von Ethylsecodion (Formel III) mittels Oxidoreduktase SEQ ID NO:5

Für die Reduktion von Ethylsecodion (Formel III) wurde in einem Reaktionsgefäß ein Gemisch aus 300 µl Puffer (100 mM Triethanolamin, pH = 7, 2 mM MgCl₂), 1,2 ml 4-Methyl-2-pentanol, 0,12 mg NADP, 150 mg Ethylsecodion (Formel III) und 0,6 ml Enzymsuspension Oxidoreduktase SEQ ID NO:5 (siehe Beispiel 3) für 72 h bei Raumtemperatur unter ständiger Durchmischung inkubiert. Nach 72 h waren >90 % des eingesetzten Ethylsecodions (Formel III) zur Hydroxyverbindung reduziert.

## Patentansprüche

1. Verfahren zur enantioselektiven enzymatischen Reduktion von Secodionderivaten der allgemeinen Formel I 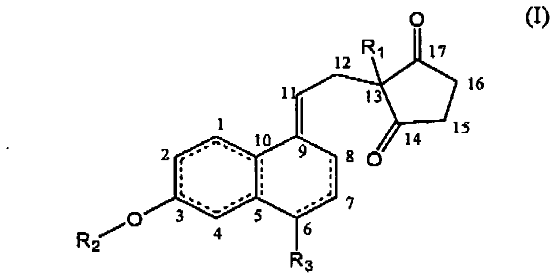 worin
R₁ Wasserstoff oder eine C₁-C₄-Alkylgruppe ist,
R₂ Wasserstoff, eine C₁-C₈-Alkylgruppe oder eine im Stand der Technik bekannte OH-Schutzgruppe, wie ein Ester, ist,
R₃ Wasserstoff, eine Methylgruppe oder ein Halogenid ist,
das Strukturelement 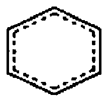 einen Benzolring oder einen C₆-Ring mit 0, 1 oder 2 C-C-Doppelbindungen repräsentiert, an den Positionen 6/7 oder 7/8 gegebenenfalls eine Doppelbindung enthalten ist und
der Kohlenstoff an den Positionen 1, 2, 4, 5, 6, 7, 8, 9, 11, 12 und 16 unabhängig mit Wasserstoff, einer C₁-C₄-Alkylgruppe, einem Halogenid oder einer Phenylgruppe substituiert ist,
wobei das Secodionderivat mit einer Oxidoreduktase/Dehydrogenase in Gegenwart von NADH oder NADPH als Cofactor reduziert wird,
**dadurch gekennzeichnet, dass** das Secodionderivate in einer Konzentration von ≥ 10 g/l im Reaktionsansatz eingesetzt wird und der durch die Oxidoreduktase/Dehydrogenase gebildete oxidierte Cofactor NAD oder NADP kontinuierlich regeneriert wird.

2. Verfahren nach Anspruch 1, **dadurch gekennzeichnet, dass** die Oxidoreduktase/Dehydrogenase
a) eine Aminosäuresequenz aufweist, bei welcher mindestens 50% der Aminosäuren mit jenen der Aminosäuresequenz SEQ ID NO:1, SEQ ID NO:2, SEQ ID NO:3, SEQ ID NO:4 oder SEQ ID NO:5 identisch sind,
b) von der Nukleinsäuresequenz SEQ ID NO:6, SEQ ID NO:7, SEQ ID NO:8, SEQ ID NO:9 oder SEQ ID NO:10 codiert wird oder
c) von einer Nukleinsäuresequenz codiert wird, die mit SEQ ID NO:6, SEQ ID NO:7, SEQ ID NO:8, SEQ ID NO:9 oder SEQ ID NO:10 unter stringenten Bedingungen hybridisiert.

3. Verfahren nach Anspruch 1, **dadurch gekennzeichnet, dass** die Oxidoreduktase/Dehydrogenase eine Länge von 230 bis 260 Aminosäuren aufweist und eine oder mehrere der Partialsequenzen, ausgewählt aus der Gruppe bestehend aus den Sequenzen SEQ ID NO:18 bis SEQ ID NO:42; umfasst.

4. Verfahren nach einem der Ansprüche 1 bis 3, **dadurch gekennzeichnet, dass** der oxidierte Cofactor NAD oder NADP durch Oxidation eines Alkohols regeneriert wird.

5. Verfahren nach Anspruch 4, **dadurch gekennzeichnet, dass** zur Cofaktorregenerierung ein sekundärer Alkohol mit der allgemeinen Formel R_{X}R_{Y}CHOH verwendet wird, wobei R_{X} und R_{Y} unabhängig voneinander eine verzweigte oder unverzweigte C₁-C₈-Alkylgruppe sind und C_{insgesamt} ≥ 3.

6. Verfahren nach Anspruch 5, **dadurch gekennzeichnet, dass** zur Cofaktorregenerierung ein Alkohol aus der Gruppe bestehend aus 2-Propanol, 2-Butanol, 2-Pentanol, 4-Methyl-2-pentanol, 2-Hexanol, 2-Heptanol, 5-Methyl-2-hexanol, Cyclohexanol oder 2-Octanol verwendet wird.

7. Verfahren nach einem der Ansprüche 1 bis 3, **dadurch gekennzeichnet, dass** zur Regenerierung des Cofaktors zusätzlich eine Oxidoreduktase/ Dehydrogenase zugesetzt wird.

8. Verfahren nach einem der Ansprüche 1 bis 7, **dadurch gekennzeichnet, dass** der TTN, d.h. total turn over number = mol reduziertes Secodionderivate / mol eingesetzter Cofactor, ≥10³ ist.

9. Verfahren nach einem der Ansprüche 1 bis 8, **dadurch gekennzeichnet, dass** es in einem wässrigen organischen Zweiphasensystem durchgeführt wird.

10. Verfahren nach einem der Ansprüche 1 bis 9, **dadurch gekennzeichnet, dass** zusätzlich ein organisches Lösungsmittel, vorzugsweise Diethylether, tertiär-Butylmethylether, Diisopropylether, Dibutylether, Ethylacetat, Butylacetat, Heptan, Hexan, Toluol, Dichlormethan, Cyclohexan oder Gemische davon, eingesetzt wird.

11. Verfahren nach Anspruch 10, **dadurch gekennzeichnet, dass** als organisches Lösungsmittel Diethylether, tertiär-Butylmethylether, Diisopropylether, Dibutylether, Ethylacetat, Butylacetat, Heptan, Hexan, Toluol, Dichlormethan, Cyclohexan oder ein Gemisch davon eingesetzt wird.

12. Verfahren nach einem der Ansprüche 1 bis 11, **dadurch gekennzeichnet, dass** das Secodionderivat in einer Menge von 10g/l bis 500 g/l, bezogen auf das Gesamtvolumen, bevorzugt von 25 g/l bis 300 g/l, besonders bevorzugt von 50g/l bis 200g/l, im Reaktionsansatz eingesetzt wird.

13. Verfahren nach Anspruch 12, **dadurch gekennzeichnet, dass** das Secodionderivat in einer Menge von 25 g/l bis 300 g/l, bezogen auf das Gesamtvolumen, im Reaktionsansatz eingesetzt wird.

14. Verfahren nach Anspruch 13, **dadurch gekennzeichnet, dass** das Secodionderivat in einer Menge von 50g/l bis 200g/l, bezogen auf das Gesamtvolumen, im Reaktionsansatz eingesetzt wird.

15. Verfahren nach einem der Ansprüche 1 bis 14, **dadurch gekennzeichnet, dass** als Secodionderivat 13-Ethyl-3-methoxy-8,14-seco-gona-1,3,5(10),9(11)-tetraen-14,17-dion, d.h. Ethylsecodion - Formel III, eingesetzt wird.

16. Verfahren nach einem der Ansprüche 1 bis 14, **dadurch gekennzeichnet, dass** als Secodionderivat 13-Methyl-3-methoxy-8,14-seco-gona-1,3,5(10),9(11)-tetraen-14,17-dion, d.h. Methylsecodion - Formel III, eingesetzt wird.

## Claims

1. A process for the enantioselective enzymatic reduction of secodione derivatives of general formula I 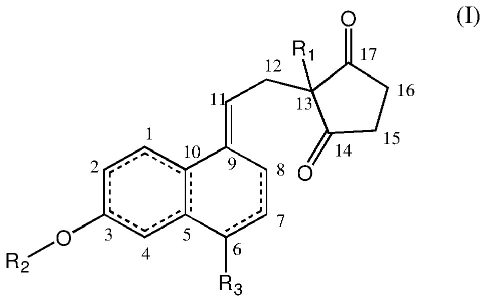 wherein
R₁ is hydrogen or a C₁-C₄ alkyl group,
R₂ is hydrogen, a C₁-C₈ alkyl group or a protective group for OH known in prior art, such as an ester,
R₃ is hydrogen, a methyl group or a halide,
the structural element 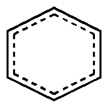 represents a benzene ring or a C₆ ring having 0, 1 or 2 C-C double bonds,
a double bond is optionally included at positions 6/7 or 7/8, and
the carbon at positions 1, 2, 4, 5, 6, 7, 8, 9, 11, 12 and 16 is independently substituted with hydrogen, a C₁-C₄ alkyl group, a halide or a phenyl group,
wherein the secodione derivative is reduced with an oxidoreductase/dehydrogenase in the presence of NADH or NADPH as a cofactor,
**characterized in that** the secodione derivative is used in the reaction batch at a concentration of ≥ 10 g/l and the oxidized cofactor NAD or NADP formed by the oxidoreductase/dehydrogenase is regenerated continuously.

2. The process according to claim 1, **characterized in that** the oxidoreductase/dehydrogenase
a) comprises an amino acid sequence in which at least 50% of the amino acids are identical to those of amino acid sequence SEQ ID NO:1, SEQ ID NO:2, SEQ ID NO:3, SEQ ID NO:4 or SEQ ID NO:5,
b) is encoded by the nucleic acid sequence SEQ ID NO:6, SEQ ID NO:7, SEQ ID NO:8, SEQ ID NO:9 or SEQ ID NO:10, or
c) is encoded by a nucleic acid sequence which hybridizes to SEQ ID NO:6, SEQ ID NO:7, SEQ ID NO:8, SEQ ID NO:9 or SEQ ID NO:10 under stringent conditions.

3. The process according to claim 1, **characterized in that** the oxidoreductase/dehydrogenase has a length of from 230 to 260 amino acids and comprises one or several of the partial sequences selected from the group consisting of sequences SEQ ID NO:18 to SEQ ID NO:42;

4. The process according to any of claims 1 to 3, **characterized in that** the oxidized cofactor NAD or NADP is regenerated by oxidation of an alcohol.

5. The process according to claim 4, **characterized in that** a secondary alcohol having the general formula R_{X}R_{Y}CHOH is used for cofactor regeneration, wherein R_{X} and R_{Y} independently of each other are a branched or unbranched C₁-C₈ alkyl group and Cₜₒₜₐₗ ≥ 3.

6. The process according to claim 5, **characterized in that** an alcohol from the group consisting of 2-propanol, 2-butanol, 2-pentanol, 4-methyl-2-pentanol, 2-hexanol, 2-heptanol, 5-methyl-2-hexanol, cyclohexanol or 2-octanol is used for cofactor regeneration.

7. The process according to any of claims 1 to 3, **characterized in that** an oxidoreductase/dehydrogenase is additionally added for the regeneration of the cofactor.

8. The process according to any of claims 1 to 7, **characterized in that** the TTN, i.e., total turn over number = mol of reduced secodione derivative / mol of cofactor used, is ≥10³.

9. The process according to any of claims 1 to 8, **characterized in that** it is carried out in an aqueous organic two-phase system.

10. The process according to any of claims 1 to 9, **characterized in that** an organic solvent, preferably diethyl ether, tertiary butyl methyl ether, diisopropyl ether, dibutyl ether, ethyl acetate, butyl acetate, heptane, hexane, toluene, dichloromethane, cyclohexane or mixtures thereof, is additionally used.

11. The process according to claim 10, **characterized in that** diethyl ether, tertiary butyl methyl ether, diisopropyl ether, dibutyl ether, ethyl acetate, butyl acetate, heptane, hexane, toluene, dichloromethane, cyclohexane or a mixture thereof, is used as the organic solvent.

12. The process according to any of claims 1 to 11, **characterized in that** the secodione derivative is used in the reaction batch in an amount of from 10 g/l to 500 g/l, based on the total volume, preferably from 25 g/l to 300 g/l, particularly preferably from 50 g/l to 200 g/l.

13. The process according to claim 12, **characterized in that** the secodione derivative is used in the reaction batch in an amount of from 25 g/l to 300 g/l, based on the total volume.

14. The process according to claim 13, **characterized in that** the secodione derivative is used in the reaction batch in an amount of from 50 g/l to 200 g/l, based on the total volume.

15. The process according to any of claims 1 to 14, **characterized in that** 13-ethyl-3-methoxy-8,14-seco-gona-1,3,5(10),9(11)-tetraene-14,17-dione, i.e., ethyl secodione - Formula III, is used as the secodione derivative.

16. The process according to any of claims 1 to 14, **characterized in that** 13-methyl-3-methoxy-8,14-seco-gona-1,3,5(10),9(11)-tetraene-14,17-dione, i.e., methyl secodione - Formula II, is used as the secodione derivative.

## Revendications

1. Procédé de réduction enzymatique énantiosélective de dérivés de sécodion de formule générale I 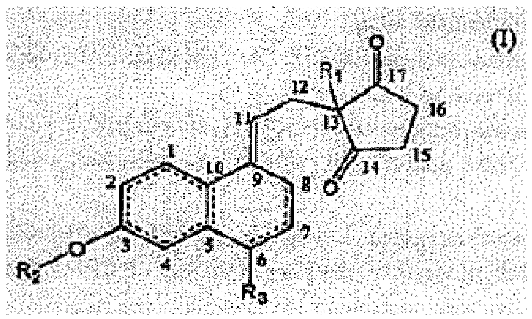 dans laquelle
R₁ représente l'hydrogène ou un groupe alkyle en C₁ à C₄, R₂ représente l'hydrogène, un groupe alkyle en C₁ à C₈ ou un groupe de protection OH connu dans l'état de la technique, par exemple un ester,
R₃ représente l'hydrogène, un groupe méthyle ou un halogénure,
l'élément structurel 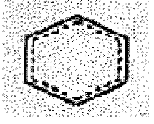 étant un cycle de benzène ou un cycle en C₆ comptant 0, 1 ou 2 doubles liaisons C-C,
une double liaison étant éventuellement prévue en positions 6/7 ou 7/8 et l'atome de carbone des positions 1, 2, 4, 5, 6, 7, 8, 9, 11, 12 et 16 étant substitué indépendamment avec l'hydrogène, un groupe alkyle en C₁ à C₄, un halogénure ou un groupe phényle,
le dérivé de sécodion étant réduit par une oxydoréductase-déshydrogénase en présence de NADH ou de NADPH comme cofacteur,
**caractérisé en ce que**
le dérivé de sécodion est utilisé à une concentration ≥ 10 g/l dans le milieu de réaction et le cofacteur oxydé NAD ou NADP formé par l'oxydoréductase-déshydrogénase est régénéré en continu.

2. Procédé selon la revendication 1, **caractérisé en ce que** l'oxydoréductase-déshydrogénase
a) présente une séquence d'acides aminés dont au moins 50 % des acides aminés sont identiques à l'une des séquences d'acides aminés SEQ ID NO:1, SEQ ID NO:2, SEQ ID NO:3, SEQ ID NO:4 ou SEQ ID NO:5,
b) est codé par la séquence d'acides nucléiques SEQ ID NO:6, SEQ ID NO:7, SEQ ID NO:8, SEQ ID NO:9 ou SEQ ID NO:10 ou
c) est codé par une séquence d'acides nucléiques qui s'hybride avec la SEQ ID NO:6, SEQ ID NO:7, SEQ ID NO:8, SEQ ID NO:9 ou la SEQ ID NO:10 en conditions stringentes.

3. Procédé selon la revendication 1, **caractérisé en ce que** l'oxydoréductase-déshydrogénase présente une longueur de 230 à 260 acides aminés et comporte une ou plusieurs des séquences partielles sélectionnées dans l'ensemble constitué des séquences SEQ ID NO:18 à SEQ ID NO:42 ;

4. Procédé selon l'une des revendications 1 à 3, **caractérisé en ce que** le cofacteur oxydé NAD ou NADP est régénéré par oxydation d'un alcool.

5. Procédé selon la revendication 4, **caractérisé en ce que** pour la régénération du cofacteur, on utilise un alcool secondaire de formule générale RₓR_{y}CHOH dans laquelle Rₓ et R_{y} représentent indépendamment l'un de l'autre un groupe alkyle en C₁ à C₈ ramifié ou non ramifié et C_{global} ≥ 3.

6. Procédé selon la revendication 5, **caractérisé en ce qu'**il utilise pour la régénération du cofacteur un alcool sélectionné dans l'ensemble constitué du 2-propanol, du 2-butanol, du 2-pentanol, du 4-méthyl-2-pentanol, du 2-hexanol, du 2-heptanol, du 5-méthyl-2-hexanol, du cyclohexanol et du 2-octanol.

7. Procédé selon l'une des revendications 1 à 3, **caractérisé en ce que** pour la régénération du cofacteur, on ajoute en supplément une oxydoréductase-déshydrogénase.

8. Procédé selon l'une des revendications 1 à 7, **caractérisé en ce que** le TTN, c'est-à-dire le nombre total de renouvellements = moles de dérivés de sécodion réduit/moles de cofacteur utilisé ≥ 10³.

9. Procédé selon l'une des revendications 1 à 8, **caractérisé en ce qu'**il est conduit dans un système aqueux organique biphasique.

10. Procédé selon l'une des revendications 1 à 9, **caractérisé en ce qu'**on y utilise en supplément un solvant organique, de préférence l'éther de diéthyle, un éther tertiaire de butyle et de méthyle, l'éther de diisopropyle, l'éther de dibutyle, l'acétate d'éthyle, l'acétate de butyle, l'heptane, l'hexane, le toluène, le dichlorométhane, le cyclohexane ou un de leurs mélanges.

11. Procédé selon la revendication 10, **caractérisé en ce qu'**on y utilise comme solvant organique l'éther de diéthyle, un éther tertiaire de butyle et de méthyle, l'éther de diisopropyle, l'éther de dibutyle, l'acétate d'éthyle, l'acétate de butyle, l'heptane, l'hexane, le toluène, le dichlorométhane, le cyclohexane ou un de leurs mélanges.

12. Procédé selon l'une des revendications 1 à 11, **caractérisé en ce que** le dérivé de sécodion est utilisé en quantité de 10 g/l à 500 g/l par rapport au volume total, de préférence de 25 g/l à 300 g/l et de façon particulièrement préférable de 50 g/l à 200 g/l.

13. Procédé selon la revendication 12, **caractérisé en ce que** le dérivé de sécodion est utilisé en quantité de 25 g/l à 300 g/l par rapport au volume total du milieu de réaction.

14. Procédé selon la revendication 13, **caractérisé en ce que** le dérivé de sécodion est utilisé en quantité de 50 g/l à 200 g/l par rapport au volume total du milieu de réaction.

15. Procédé selon l'une des revendications 1 à 14, **caractérisé en ce qu'**il utilise comme dérivé de sécodion le 13-éthyl-3-méthoxy-8,14-seco-gona-1,3,5(10),9(11)-tétraène-14,17-dione, c'est-à-dire l'éthylsécodion de formule III.

16. Procédé selon l'une des revendications 1 à 14, **caractérisé en ce qu'**il utilise comme dérivé de sécodion le 13-méthyl-3-méthoxy-8,14-seco-gona-1,3,5(10),9(11)-tétraène-14,17-dione, c'est-à-dire le méthylsécodion de formule II.
